(19) Europäisches Patentamt
European Patent Office
Office européen des brevets

(11) EP 3 578 983 A1

(12) **EUROPEAN PATENT APPLICATION**
published in accordance with Art. 153(4) EPC

(43) Date of publication:
**11.12.2019 Bulletin 2019/50**

(21) Application number: **18748415.9**

(22) Date of filing: **01.02.2018**

(51) Int Cl.:
***G01N 33/574*** *(2006.01)* ***G01N 33/564*** *(2006.01)*

(86) International application number:
**PCT/JP2018/003394**

(87) International publication number:
**WO 2018/143336 (09.08.2018 Gazette 2018/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **03.02.2017 JP 2017018215**

(71) Applicants:
- **Sumitomo Chemical Company, Limited**
  **Tokyo 104-8260 (JP)**
- **Educational Foundation Ohtani Gakuen**
  **Osaka-shi, Osaka 545-0041 (JP)**

(72) Inventors:
- **HIGASHI, Kiyoshi**
  **Osaka-shi**
  **Osaka 554-8558 (JP)**
- **SAITO, Koichi**
  **Osaka-shi**
  **Osaka 554-8558 (JP)**
- **YAMADA, Keita**
  **Tondabayashi-shi**
  **Osaka 584-8540 (JP)**

(74) Representative: **Vossius & Partner**
**Patentanwälte Rechtsanwälte mbB**
**Siebertstrasse 3**
**81675 München (DE)**

# (54) PANCREATIC CANCER DETECTION METHOD

(57) An object is to provide a test method for pancreatic cancer, and further to provide a test method for pancreatic cancer capable of detecting pancreatic cancer even at earlier stages. The objects are solved by a method for examining the likelihood of having pancreatic cancer, the method including (1) measuring the amount of at least one anti-carbohydrate antibody selected from the group consisting of anti-3-sialyllactose carbohydrate antibody and anti-(LN)3 carbohydrate antibody in a biological sample obtained from a subject, wherein the anti-3-sialyllactose carbohydrate antibody is an antibody capable of binding to a carbohydrate represented by Neu5Acα (2→3) Galβ (1→4) Glc or an antibody recognizing the carbohydrate, and wherein the anti-(LN)3 carbohydrate antibody is an antibody capable of binding to a carbohydrate represented by Galβ1 → 3GlcNAcβ1 → 3Galβ1 → 4GlcNAc or an antibody recognizing the carbohydrate.

EP 3 578 983 A1

**Description**

Technical Field

The present invention relates to a test method for pancreatic cancer, a test agent for pancreatic cancer, and the like.

Background Art

Pancreatic cancer is the fifth most common cause of death from cancer in men and the fourth in women (2014), with the number being on the rise. The difficulty of early detection is considered to be one of the reasons for high fatalities from pancreatic cancer. Pancreatic cancer does not cause distinctive symptoms in the early stages, making early detection difficult. By the time of diagnosis, pancreatic cancer is often at an advanced stage and poorly treated.

Carbohydrate antigens and antibodies against them are reported as detection indicators for various cancers. For example, a study reports a method using carbohydrate antigen 19-9 (CA19-9), which is one of the carbohydrate antigens, as a test method for pancreatic cancer (NPL 1).

Citation List

Non-patent Literature

NPL 1: Erxi W., Shuang Z., Kruttika B., Qingyong M.: CA 19-9 and pancreatic cancer. Clin Adv Hematol Oncol. 2013 11 (1): 53-55.

Summary of Invention

Technical Problem

An object of the present invention is to provide a test method for pancreatic cancer. Furthermore, an object of the present invention is to provide a test method for pancreatic cancer that is even capable of detecting pancreatic cancer at early stages.

Solution to Problem

The present invention is based on the findings that the level of serum anti-carbohydrate antibodies (anti-3-sialyllactose carbohydrate antibody and anti-(LN)3 carbohydrate antibody) tends to be lower in pancreatic cancer patients than in healthy individuals, and that the likelihood of having pancreatic cancer can be determined by using the anti-carbohydrate antibody level as an indicator.

The present invention includes the following aspect as one aspect.

Item 1. A method for examining the likelihood of having pancreatic cancer,

the method comprising (1) measuring the amount or concentration of at least one anti-carbohydrate antibody selected from the group consisting of anti-3-sialyllactose carbohydrate antibody and anti-(LN)3 carbohydrate antibody in a biological sample obtained from a subject,
wherein the anti-3-sialyllactose carbohydrate antibody is an antibody capable of binding to a carbohydrate represented by Neu5Ac$\alpha$ (2$\rightarrow$3) Gal$\beta$ (1$\rightarrow$4) Glc or an antibody recognizing the carbohydrate, and
the anti-(LN)3 carbohydrate antibody is an antibody capable of binding to a carbohydrate represented by Gal$\beta$1 $\rightarrow$ 3GlcNAc$\beta$1 $\rightarrow$ 3Gal$\beta$1 $\rightarrow$ 4GlcNAc or an antibody recognizing the carbohydrate.

Item 2. The method according to Item 1, wherein step (1) comprises

(1a) bringing at least one compound selected from the group consisting of compound 1 containing a 3-sialyllactose carbohydrate structure and compound 2 containing an (LN)-3 carbohydrate structure into contact with the biological sample; and
(1b) measuring the amount of an anti-carbohydrate antibody bound to the at least one compound.

Item 3. The method according to Item 1 or 2, further comprising

(2a) determining that the subject has a high likelihood of having pancreatic cancer when the value of the amount or concentration of the at least one anti-carbohydrate antibody measured in step (1) is less than or equal to a predetermined cutoff value.

Item 4. The method according to Item 3, wherein the cutoff value is an average value, a percentile value, or a minimum value of the amounts or concentrations of the at least one anti-carbohydrate antibody in biological samples obtained from subjects who are not affected by pancreatic cancer.

Item 5. The method according to Item 1 or 2, further comprising

(2c) tentatively determining that the subject has a high likelihood of having pancreatic cancer when the value of the amount or concentration of the at least one anti-carbohydrate antibody measured in step (1) is less than or equal to a predetermined cutoff value;
(3c) measuring the amount or concentration of a pancreatic-cancer-carbohydrate marker in the biological sample, and tentatively determining that the subject has a high likelihood of having pancreatic cancer when the value of the measured amount or concentration of the pancreatic-cancer-carbohydrate marker is equal to or more than a predetermined cutoff value; and
(3d) determining that the subject has a high likelihood of having pancreatic cancer on the basis of the tentative determination in step (2c) and the tentative determination in step (3c).

Item 6. The method according to Item 5, wherein step (3d) comprises determining that the subject has a high likelihood of having pancreatic cancer when the subject has been tentatively determined to have a high likelihood of having pancreatic cancer in either the tentative determination in step (2c) or the tentative determination in step (3c), or both.

Item 7. The method according to Item 5 or 6, wherein the cutoff value for the amount or concentration of the at least one anti-carbohydrate antibody is an average value, a percentile value, or a minimum value of the amounts or concentrations of the at least one anti-carbohydrate antibody in biological samples obtained from subjects who are not affected by pancreatic cancer.

Item 8. The method according to any one of Items 5 to 7, wherein the pancreatic-cancer-carbohydrate marker is at least one member selected from the group consisting of CA19-9 and DUPAN-2.

Item 9. The method according to Item 8, wherein the pancreatic-cancer-carbohydrate marker is CA19-9.

Item 10. The method according to any one of Items 1 to 9, wherein the at least one anti-carbohydrate antibody is IgG.

Item 11. The method according to any one of Items 1 to 10, wherein the biological sample is a body fluid or a sample derived from a body fluid.

Item 12. The method according to Item 11, wherein the body fluid is at least one member selected from the group consisting of whole blood, serum, and plasma.

Item 13. The method according to any one of Items 1 to 12, wherein the pancreatic cancer to be detected is at a stage of 0 to 2.

Item 14. A test agent for pancreatic cancer comprising at least one compound selected from the group consisting of compound 1 containing a 3-sialyllactose carbohydrate structure and compound 2 containing an (LN)3 carbohydrate structure,
wherein the 3-sialyllactose carbohydrate structure is represented by Neu5Acα (2→3) Galβ (1→4) Glc, and the (LN)3 carbohydrate structure is represented by Galβ1 → 3GlcNAcβ1 → 3Galβ1 → 4GlcNAc.
The present invention also includes the following aspect as another aspect.

Item 15. A method for aiding in determining the likelihood of having pancreatic cancer,
the method comprising (1) measuring the amount or concentration of at least one anti-carbohydrate antibody selected from the group consisting of anti-3-sialyllactose carbohydrate antibody and anti-(LN)3 carbohydrate antibody in a biological sample obtained from a subject.

Item 16. A method for measuring the amount or concentration of an anti-carbohydrate antibody in a subject, the method comprising (1) measuring the amount or concentration of at least one anti-carbohydrate antibody selected from the group consisting of anti-3-sialyllactose carbohydrate antibody and anti-(LN)3 carbohydrate antibody in a biological sample obtained from a subject.

Item 17. The method according to Item 16, wherein in step (1), the amount or concentration of only one anti-carbohydrate antibody selected from the group consisting of anti-3-sialyllactose carbohydrate antibody and anti-(LN)3 carbohydrate antibody is measured as the anti-carbohydrate antibody.

Item 18. The method according to Item 16 or 17, wherein step (1) comprises

immobilizing on a solid phase at least one compound selected from the group consisting of compound 1 containing a 3-sialyllactose carbohydrate structure and compound 2 containing an (LN)3 carbohydrate structure,
bringing the at least one compound immobilized on the solid phase into contact with a biological sample obtained from a subject,
washing the solid phase on which the at least one compound that has been brought into contact with the biological sample is immobilized with a solution containing trishydroxymethylaminomethane and/or an ether-type nonionic surfactant,
adding a blocking solution that contains bovine serum albumin and that further contains trishydroxymethylaminomethane and/or an ether-type nonionic surfactant to the washed solid phase, and
measuring the amount or concentration of an antibody bound to the at least one compound immobilized on the solid phase.

Item 19. A method for diagnosing pancreatic cancer in a subject, the method comprising

(1) measuring the amount or concentration of at least one anti-carbohydrate antibody selected from the group consisting of anti-3-sialyllactose carbohydrate antibody and anti-(LN)3 carbohydrate antibody in a biological sample obtained from a subject,
(2a) determining that the subject has a high likelihood of having pancreatic cancer when the value of the amount or concentration of the at least one anti-carbohydrate antibody measured in step (1) is less than or equal to a predetermined cutoff value, and
(3a) applying a method for diagnosing pancreatic cancer to the subject who has been determined to have a high likelihood of having pancreatic cancer in step (2a).

Item 20. The method according to Item 19, wherein the method for diagnosing pancreatic cancer applied in step (3a) is at least one method selected from the group consisting of biopsy, PET, CT, and ultrasonography.

Item 21. A method for diagnosing and treating pancreatic cancer in a subject, the method comprising

(1) measuring the amount or concentration of at least one anti-carbohydrate antibody selected from the group consisting of anti-3-sialyllactose carbohydrate antibody and anti-(LN)3 carbohydrate antibody in a biological sample obtained from a subject,
(2a) determining that the subject has a high likelihood of having pancreatic cancer when the value of the amount or concentration of the at least one anti-carbohydrate antibody measured in step (1) is less than or equal to a predetermined cutoff value, or (3b) determining that the subject has a high likelihood of having pancreatic cancer when the value of the amount or concentration of the at least one anti-carbohydrate antibody measured in step (1) (a level of the anti-carbohydrate antibody tested) is less than or equal to the predetermined cutoff value, and applying a method for diagnosing pancreatic cancer to the subject who has been determined to have a high likelihood of having pancreatic cancer, and
(4) performing pancreatic cancer treatment on the subject determined to have a high likelihood of having pancreatic cancer in step (2a) or the subject diagnosed with pancreatic cancer in step (3b).

Item 22. A compound for use in a test agent for examining the likelihood of having pancreatic cancer, the compound being at least one compound selected from the group consisting of compound 1 containing a 3-sialyllactose carbohydrate structure and compound 2 containing an (LN)3 carbohydrate structure.

Item 23. Use of a compound in examining the likelihood of having pancreatic cancer, the compound being at least one compound selected from the group consisting of compound 1 containing a 3-sialyllactose carbohydrate structure

and compound 2 containing an (LN)3 carbohydrate structure.

Item 24. Use of a compound in producing a test agent for examining the likelihood of pancreatic cancer, the compound being at least one compound selected from the group consisting of compound 1 containing a 3-sialyllactose carbohydrate structure and compound 2 containing an (LN)3 carbohydrate structure.

Advantageous Effects of Invention

The present invention provides a test method for pancreatic cancer. The test method according to the present invention can examine the likelihood of having pancreatic cancer (preferably, pancreatic cancer at early stages) with higher sensitivity to even detect pancreatic cancer earlier.

Brief Description of the Drawings

Fig. 1 shows anti-carbohydrate antibody (anti-3-sialyllactose carbohydrate antibody and anti-(LN)3 carbohydrate antibody) concentrations (anti-carbohydrate antibody levels) in the serum of healthy subject specimens and pancreatic cancer patient specimens measured and calculated in Reference Example 1(b). The vertical axis in each graph represents the anti-carbohydrate antibody level (unit: fluorescence intensity value/amount of IgG ($\mu$g)). In the horizontal axis, "healthy" represents the results of healthy subject specimens, "pancreatic cancer" represents the results of pancreatic cancer patient specimens, and "N" represents the number of specimens.

Fig. 2 shows the results of the development of pancreatic cancer at each stage determined in Example 1 and Comparative Example 1. The vertical axis represents the determination indicator. The horizontal axis represents the pancreatic cancer stage. The horizontal line in each graph indicates the predetermined cutoff value. The fractions in each graph indicate sensitivity, i.e., the number of specimens that are determined to have a high likelihood of having pancreatic cancer/the number of pancreatic cancer patient specimens.

Fig. 3 shows anti-3-sialyllactose carbohydrate antibody concentrations in the serum of healthy subject specimens and pancreatic cancer patient specimens measured in Reference Example 2 and Example 2.

Fig. 4 shows an ROC curve with respect to the anti-3-sialyllactose carbohydrate antibody concentrations ($\mu$g/mL) in the serum of healthy subject specimens and pancreatic cancer patient specimens measured in Reference Example 2 and Example 2. The vertical axis represents sensitivity. The horizontal axis represents a value obtained by subtracting the percentage of specificity from 100% (100% - specificity (%)).

Description of Embodiments

In the present specification, the expressions "containing" and "including" include the concepts of "containing," "including," "consisting essentially of," and "consisting of."

1. Method for Examining the Likelihood of Having Pancreatic Cancer

One aspect of the present invention relates to a method for examining the likelihood of having pancreatic cancer, which includes (1) measuring the amount or concentration of at least one anti-carbohydrate antibody selected from the group consisting of anti-3-sialyllactose carbohydrate antibody and anti-(LN)3 carbohydrate antibody in a biological sample obtained from a subject (step (1)) (this method may be referred to as "the test method of the present invention" in this specification). The following describes the method.

1-1. Step (1)

Pancreatic cancer to be detected is not particularly limited and includes pancreatic cancer of all types at any stage. Examples of the type of pancreatic cancer to be detected include pancreatic ductal carcinoma, pancreatic endocrine tumors, intraductal papillary mucinous neoplasm, mucinous cystic neoplasm, and acinar cell carcinoma, with pancreatic ductal carcinoma being preferable. The stages of pancreatic cancer to be detected include stage 0, stage 1, stage 2, stage 3, and stage 4 (stage 4a and stage 4b) in order from the earliest stage. The test method of the present invention is useful in its capability of even detecting pancreatic cancer at early stages. The test method of the present invention can detect pancreatic cancer at stage 2 or earlier, in particular pancreatic cancer at stage 1 or stage 2 with higher accuracy than known methods.

From this viewpoint, the stage of pancreatic cancer to be detected is preferably stages 0 to 3, more preferably

stages 0 to 2, and still more preferably stages 1 to 2.

The "subject" refers to a target organism of the test method of the present invention, and the species of the subject is not particularly limited. Examples of species of the subject include a variety of mammals, such as humans, monkeys, mice, rats, dogs, cats, and rabbits, with humans being preferable.

The state of pancreatic cancer in a subject is not particularly limited. Examples of the subject include a subject about whom it is unknown whether the subject has pancreatic cancer, a subject who has no pancreatic cancer history, a subject who has a pancreatic cancer history and has been treated for pancreatic cancer, a subject who has been determined to have (or not have) pancreatic cancer by another determination method.

The biological sample is not particularly limited as long as the sample can contain an anti-carbohydrate antibody. Examples of biological samples include body fluids, such as whole blood, serum, plasma, saliva, spinal fluid, joint fluid, urine, tissue fluid, sweat, tear, and saliva, and samples derived from these body fluids. A sample derived from body fluid is not particularly limited as long as it is prepared from body fluid. Examples include samples prepared by, for example, concentrating and/or purifying an antibody contained in body fluid from the body fluid (preferably an anti-carbohydrate antibody, and more preferably at least one anti-carbohydrate antibody selected from the group consisting of anti-3-sialyllactose carbohydrate antibody and anti-(LN)3 carbohydrate antibody). Preferable body fluids include whole blood, serum, and plasma. These biological samples may be used singly, or in a combination of two or more.

A biological sample can be obtained from a subject by a method known to those skilled in the art. For example, whole blood can be obtained by, for example, blood collection using a syringe. It is preferred that medical staff such as doctors and nurses perform blood collection. Serum is a portion obtained by removing blood cells and particular blood coagulation factors from blood, and can be obtained, for example, as a supernatant after coagulating blood. Plasma is a portion obtained by removing blood cells from blood, and can be obtained, for example, as a supernatant when blood is centrifuged without coagulating the blood.

3-sialyllactose carbohydrate is represented by Neu5Ac$\alpha$ (2$\rightarrow$3) Gal$\beta$ (1$\rightarrow$4) Glc, and is more specifically a carbohydrate in which the hydroxy group at position 2 of neuraminic acid (Neu5Ac) is linked to the hydroxy group at position 3 of galactose (Gal) by an $\alpha$-glycosidic bond, and the hydroxy group at position 1 of the galactose (Gal) is linked to the hydroxy group at position 4 of glucose (Glc) by a $\beta$-glycosidic bond. An example of the structural formula of 3-sialyllactose carbohydrate is the following formula (A), wherein Ac represents an acetyl group.

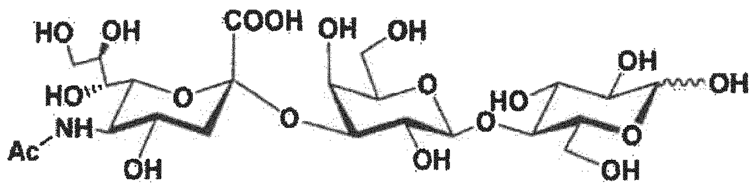

(A)

(LN)3 carbohydrate is represented by Gal$\beta$1 $\rightarrow$ 3GlcNAc$\beta$1 $\rightarrow$ 3Gal$\beta$1 $\rightarrow$ 4GlcNAc, and more specifically a carbohydrate in which the hydroxy group at position 1 of galactose (Gal, which may be referred to as "galactose A" for convenience) is linked to the hydroxy group at position 3 of N-acetylglucosamine (GlcNAc, which may be referred to as "N-acetylglucosamine A" for convenience) by a $\beta$-glycosidic bond, the hydroxy group at position 1 of N-acetylglucosamine A is linked to the hydroxy group at position 3 of another galactose, which is not galactose A, (Gal, which may be referred to as "galactose B" for convenience) by a $\beta$-glycosidic bond, and the hydroxy group at position 1 of galactose B is linked to the hydroxy group at position 4 of another N-acetylglucosamine, which is not N-acetylglucosamine A, by a $\beta$-glycosidic bond. An example of the structural formula of (LN)3 carbohydrate is the following formula (B), wherein Ac represents an acetyl group.

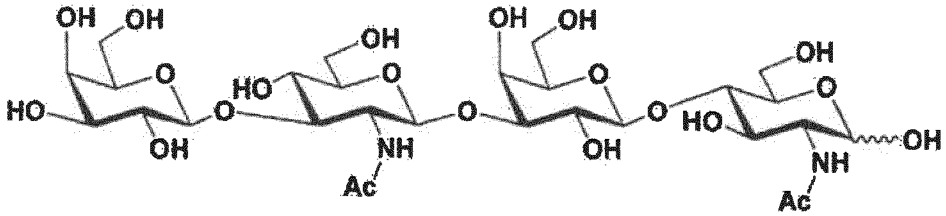

(B)

The anti-3-sialyllactose carbohydrate antibody is not particularly limited as long as the antibody is capable of (preferably specifically) binding to 3-sialyllactose carbohydrate and/or (preferably specifically) recognizes 3-sialyllactose carbohydrate. The anti-(LN)3 carbohydrate antibody is not particularly limited as long as the antibody is capable of (preferably specifically) binding to (LN)3 carbohydrate and/or (preferably) specifically recognizes (LN)3 carbohydrate. In the present specification, these antibodies may be collectively referred to as "anti-carbohydrate antibody of the present

invention."

In step (1), the amount or concentration of at least one antibody selected from the group consisting of anti-3-sialyllactose carbohydrate antibody and anti-(LN)3 carbohydrate antibody is measured. In step (1), from the standpoint of test efficiency, the amount or concentration of only one antibody selected from the group consisting of anti-3-sialyllactose carbohydrate antibody and anti-(LN)3 carbohydrate antibody is preferably measured as the anti-carbohydrate antibody.

The isotype of the anti-carbohydrate antibody of the present invention to be detected is not particularly limited. Examples of the isotype include IgG (IgG1, IgG2, IgG3, and IgG4), IgM, IgA, IgD, and IgE. Of these, for example, IgG is preferable from the standpoint of its higher accuracy in examining the likelihood of having pancreatic cancer.

In the step of measuring the amount or concentration of an antibody included in the test method of the present invention, the method for measuring the amount or concentration of an anti-carbohydrate antibody is not particularly limited as long as the method can measure the amount and/or concentration of the antibody. The method includes, for example, immunoassays. A wide range of immunoassays can be used, such as direct methods, indirect methods, homogeneous methods, heterogeneous methods, competitive methods, and non-competitive methods. More specific examples of immunoassays include ELISA (e.g., a direct method, an indirect method, a sandwich method, and a competition method), radioimmunoassay (RIA), immunoradiometric assay (IRMA), enzyme immunoassay (EIA), sandwich EIA, immunochromatography, Western blotting, immunoprecipitation, slot-blot or dot-blot assay, immunotissue staining, fluorescence immunoassay, immunoassay using an avidin-biotin or streptavidin-biotin system, and immunoassay using a surface plasmon resonance (SPR) method. These detection methods may be used singly, or in a combination of two or more.

In the step of measuring the amount or concentration of the antibody, the type of the label in a labeled object (e.g., a labeled antibody) used in detecting the antibody is not particularly limited. Examples of labels include fluorescent substances, luminescent substances, dyes, enzymes, gold colloids, and radioactive isotopes. Of these, enzyme labels, such as peroxidase and alkaline phosphatase, are preferable from the standpoint of safety, economy, detection sensitivity, etc.

Due to step (1), the amount or concentration of the anti-carbohydrate antibody of the present invention in a biological sample obtained from a subject can be measured and calculated. Details of the measurement and calculation methods are described in the following preferred embodiment of step (1).

An example of preferred embodiments of step (1) includes the steps of (1a) bringing at least one compound selected from the group consisting of compound 1 containing a 3-sialyllactose carbohydrate structure and compound 2 containing an (LN)3 carbohydrate structure into contact with a biological sample obtained from a subject (step (1a)), and (1b) measuring the amount of an antibody bound to the at least one compound (step (1b)).

Compound 1 containing a 3-sialyllactose carbohydrate structure is 3-sialyllactose carbohydrate itself or a compound containing 3-sialyllactose carbohydrate as a partial structure. In the latter case, the structure other than the 3-sialyllactose carbohydrate structure is not particularly limited, and may have antigenicity or no antigenicity. An example of a structure that has no antigenicity is one that functions as a spacer when a carbohydrate array is produced. In the case of a structure that has antigenicity, relatively many antibodies other than anti-3-sialyllactose carbohydrate antibody are expected to bind to compound 1, and this is likely to become the background. If the background is high, measurement using a compound consisting of a structure having antigenicity can be separately performed, and the obtained measurement value can be subtracted as a background value. Preferable compound 1 includes a carbohydrate having a 3-sialyllactose carbohydrate structure (e.g., with 3 to 30 sugar residues, preferably 3 to 10 sugar residues, more preferably 3 to 5 sugar residues, and still more preferably 3 sugar residues); carbohydrate polymers containing the carbohydrate having a 3-sialyllactose carbohydrate structure covalently bound to a synthetic polymer (e.g., polyacrylamide, polylysine, polystyrene, polyethylene, polyester, polyvinyl chloride, and polyurethane); glycoproteins containing the carbohydrate having a 3-sialyllactose carbohydrate structure covalently bound to serum albumin or mucin; glycopeptides containing the carbohydrate having a 3-sialyllactose carbohydrate structure covalently bound to peptides; carbohydrate dendrimers; and carbohydrates labeled using reductive amination reaction. Compound 1 for use may be one synthesized according to a known method, or a range of commercial products, such as 3'-sialyllactose-PAA (produced by GlycoTech Corporation, catalog number: 08-038).

Compound 2 containing an (LN)3 carbohydrate structure is (LN)3 carbohydrate itself or a compound containing (LN)3 carbohydrate as a partial structure. In the latter case, the structure other than the (LN)3 carbohydrate structure is not particularly limited, and may have antigenicity or no antigenicity. An example of a structure that has no antigenicity is one that functions as a spacer when a carbohydrate array is produced. In the case of a structure that has antigenicity, relatively many antibodies other than anti-(LN)3 carbohydrate antibody are expected to bind to compound 2, and this is likely to become the background. If the background is high, measurement using a compound consisting of a structure having antigenicity can be separately performed, and the obtained measurement value can be subtracted as a background value. Preferable compound 2 includes a carbohydrate having an (LN)3 carbohydrate structure (e.g., with 4 to 30 sugar residues, preferably 4 to 6 sugar residues, more preferably 4 to 5 sugar residues, and still more preferably 4 sugar

residues); carbohydrate polymers containing the carbohydrate having an (LN)3 carbohydrate structure covalently bound to a synthetic polymer (e.g., polyacrylamide, polylysine, polystyrene, polyethylene, polyester, polyvinyl chloride, and polyurethane); glycoproteins containing the carbohydrate having an (LN)3 carbohydrate structure covalently bound to serum albumin or mucin; glycopeptides containing the carbohydrates having an (LN)3 carbohydrate structure covalently bound to peptides; carbohydrate dendrimers; and carbohydrates labeled using reductive amination reaction. Compound 2 for use may be one synthesized according to a known method, or a range of commercial products, such as Galβ1 → 3GlcNAcβ1 → 3Galβ1 → 4GlcNAc-PAA (produced by GlycoTech Corporation, catalog number: 08-096).

The mode of contact in step (1a) is not particularly limited, and a suitable mode can be selected according to the type of the method for measuring the amount or concentration of the anti-carbohydrate antibody described above (e.g., various immunoassays). Examples of the contact mode include a mode in which either an antibody in a biological sample or a carbohydrate antigen (compound 1 and/or compound 2), but not both, is immobilized on a solid phase, and both are brought into contact with each other; and a mode in which the antibody and the carbohydrate antigen are brought into contact without both being not immobilized on a solid phase. Of these, from the standpoint of, for example, efficiency, preferable is the mode in which the antibody and the carbohydrate antigen are brought into contact with each other with only the carbohydrate antigen (compound 1 and/or compound 2) being immobilized on a solid phase. When either the antibody in a biological sample or the carbohydrate antigen, but not both, is immobilized on a solid phase in a contact mode in step (1a), it is preferable to wash the solid phase on which either the antibody or the carbohydrate antigen is immobilized with a solution containing trishydroxymethylaminomethane (Tris) and/or an ether-type, nonionic surfactant after immobilization.

The solid phase is not particularly limited as long as the solid phase can immobilize an antibody in a biological sample or a carbohydrate antigen (compound 1 and/or compound 2). Examples of the solid phase include plates, slides, and membranes, all of which contain polystyrene, glass, nitrocellulose, or the like as the main component. The solid phase may be coated with a component for more easily immobilizing the antibody in a biological sample or the carbohydrate antigen (compound 1 and/or compound 2), such as a reaction-facilitating compound (e.g., a compound having a reactive group or a gold colloid). The compound having a reactive group is capable of forming a covalent bond with a carbohydrate or a carbohydrate derivative, examples of which include compounds having a (1H-imidazol-1-yl) carbonyl group, a succinimidyloxycarbonyl group, an epoxy group, an aldehyde group, an amino group, a thiol group, a carboxyl group, an azide group, a cyano group, an active ester group (e.g., a 1H-benzotriazol-1-yloxycarbonyl group, a pentafluorophenyloxycarbonyl group, and a paranitrophenyloxycarbonyl group), a halogenated carbonyl group (e.g., a carbonyl chloride group, a carbonyl fluoride group, a carbonyl bromide group, and a carbonyl iodide group), or the like.

Examples of the compound having a reactive group include epoxysilane and polylysine.

When using a solid phase coated with a reaction-facilitating compound, it is preferable to block the reaction-facilitating compound with a bovine serum albumin (BSA) buffer. The blocking time is preferably 60 minutes or more.

The blocking solution preferably further contains trishydroxymethylaminomethane (Tris) and/or an ether-type nonionic surfactant.

The mode of measuring the amount or concentration of the antibody in step (1b) is not particularly limited, and a suitable mode can be selected depending on the type of the method for measuring the amount or concentration of an anti-carbohydrate antibody described above (e.g., various immunoassays). The measurement can be performed, for example, by quantifying a signal derived from the label of the labeled object used. A more specific mode of measurement is, for example, the following: a labeled antibody is brought into contact with an antibody bound to a carbohydrate antigen (compound 1 and/or compound 2), and a signal derived from the label of the bound labeled antibody is quantified.

One example of this technique is ELISA. In ELISA, a calibration curve is prepared using a reference antibody to quantify the concentration of the anti-carbohydrate antibody of the present invention. A reference antibody can be prepared, for example, from a commercially available antibody by affinity purification using a biotin-labeled carbohydrate antigen and a streptavidin-agarose resin. First, the wells of an appropriate ELISA plate are coated with a carbohydrate antigen and blocked with a BSA buffer. Second, a reference antibody or sample of different concentrations is added to the wells and allowed to stand for a predetermined period of time, followed by washing the wells. A second antibody labeled with peroxidase is added to the wells and allowed to stand for a predetermined period of time. Thereafter, the wells are washed, and a substrate of peroxidase is added to the wells, followed by allowing it to stand for a predetermined time to produce color. After a reaction-stopping agent, such as sulfuric acid, is added, absorbance is measured with a plate reader. A calibration curve is made based on the concentration of the reference antibody and its absorbance, and then the concentration of the anti-carbohydrate antibody in the sample is quantified based on the calibration curve.

The amount of the anti-carbohydrate antibody of the present invention can be calculated based on the obtained signal level. For example, in a non-competitive method, the obtained signal level can be used directly as the amount of the anti-carbohydrate antibody of the present invention. For another example, in a competitive method, because the obtained signal level has an inverse relation with the amount of the anti-carbohydrate antibody of the present invention, the amount of the anti-carbohydrate antibody of the present invention can be calculated based on the signal level determined from this relation.

The concentration of the anti-carbohydrate antibody of the present invention can also be calculated by dividing the amount of the anti-carbohydrate antibody of the present invention by the amount of the biological sample or the amount of components in the biological sample (e.g., the total amount of antibody and the total amount of antibody of the same isotype as the anti-carbohydrate antibody of the present invention to be detected).

The test method including step (1) according to the present invention can provide the value of the anti-carbohydrate antibody of a subject, which is a detection indicator for pancreatic cancer. This aids in determining the likelihood of having pancreatic cancer.

1-2. Step (2a)

In one embodiment of the test method according to the present invention, the method preferably includes (2a) determining that the subject has a high likelihood of having pancreatic cancer when the value of the amount or concentration of the anti-carbohydrate antibody of the present invention measured in step (1) is less than or equal to a predetermined cutoff value (step (2a)). As used here, the "likelihood of having pancreatic cancer" means the possibility that the subject has pancreatic cancer at the time the subject's biological sample has been obtained.

The test method including step (2a) according to the present invention can determine the likelihood of having pancreatic cancer. Additionally, because the test method of the present invention can determine the likelihood of having pancreatic cancer with higher sensitivity, the test method including step (2a) according to the present invention can more reliably determine that a subject who is truly affected by pancreatic cancer has pancreatic cancer (i.e., this can further decrease the possibility of falsely determining that such a subject does not have pancreatic cancer).

The cutoff value can be suitably determined by those skilled in the art from the standpoint of sensitivity, specificity, positive predictive value, negative predictive value, etc. For example, the cutoff value can be the average value, percentile value, or minimum value of the amounts or concentrations of the anti-carbohydrate antibody of the present invention in biological samples obtained from subjects who are not affected by pancreatic cancer. More specifically, the cutoff value can be determined, for example, by measuring the amount or concentration of the anti-carbohydrate antibody of the present invention in biological samples obtained from subjects who have pancreatic cancer and subjects who are not affected by pancreatic cancer, and performing statistical analysis on the basis of, for example, an analysis of a receiver operating characteristic (ROC) curve using the measurement values (more specifically, a method using the Youden index is an example). The cut-off value may be, for example, the 10th to 90th percentile, preferably the 30th to 70th percentile, more preferably the 40th to 60th percentile, and still more preferably the 45th to 55th percentile of the value of the amount or concentration of the anti-carbohydrate antibody of the present invention in biological samples obtained from subjects who are not affected by pancreatic cancer.

The cut-off value may be the value of the amount or concentration of the anti-carbohydrate antibody in a biological sample obtained from the same subject before a predetermined period of time. The "predetermined period of time" is not particularly limited as long as the amount or concentration of the anti-carbohydrate antibody of the present invention can change in the same subject. Examples include a period of about 1 month to 10 years, 2 months to 5 years, 3 months to 2 years, and 4 months to 1 year.

1-3. Step (2b)

Examples of modified versions of the test method of the present invention include a method that includes, instead of step (2a), (2b) determining that a subject has a high likelihood of developing pancreatic cancer in the future when the value of the amount or concentration of the anti-carbohydrate antibody of the present invention measured in step (1) is lower than the value of the amount or concentration of the anti-carbohydrate antibody of the present invention in a biological sample obtained from the same subject before a predetermined period of time (step (2b)). The test method including step (2b) determines the risk of developing pancreatic cancer in the future.

The "predetermined period of time" is not particularly limited as long as the amount or concentration of the anti-carbohydrate antibody of the present invention can change in the same subject. Examples include a period of about 1 month to 10 years, 2 months to 5 years, 3 months to 2 years, and 4 months to 1 year.

The degree of lowness is not particularly limited. For example, "low" means that the value of the amount or concentration of the anti-carbohydrate antibody of the present invention measured in step (1) is 90% or less, 70% or less, 50% or less, or 30% or less than the value of the amount or concentration of the anti-carbohydrate antibody of the present invention in a biological sample obtained from the same subject before a predetermined period of time.

1-4. Step (2c)

In another embodiment of the test method of the present invention, the method includes, instead of step (2a), (2c) tentatively determining that the subject has a high likelihood of having pancreatic cancer when the value of the

amount or concentration of the anti-carbohydrate antibody measured in step (1) is less than or equal to a predetermined cutoff value (step (2c));

(3c) measuring the amount or concentration of a pancreatic-cancer-carbohydrate marker in the biological sample, and tentatively determining that the subject has a high likelihood of having pancreatic cancer when the value of the measured amount or concentration of the pancreatic-cancer-carbohydrate marker is equal to or more than a predetermined cutoff value (step (3c)); and

(3d) determining that the subject has a high likelihood of having pancreatic cancer on the basis of the tentative determination in step (2c) and the tentative determination in step (3c) (step (3d)).

The test method including steps (2c), (3c), and (3d) examines the likelihood of having pancreatic cancer. The test method including steps (2c), (3c), and (3d) according to the present invention can determine whether a subject has pancreatic cancer at an early stage, more specifically, pancreatic cancer at stage 2 or earlier, in particular pancreatic cancer at stage 1 or 2 with higher accuracy than known methods.

For example, when a subject has been tentatively determined to have a high likelihood of having pancreatic cancer in either the tentative determination in step (2c) or the tentative determination in step (3c), or both, the subject is determined to have a high likelihood of having pancreatic cancer in step (3d). This can further reduce the possibility of falsely determining that a subject does not have pancreatic cancer despite the fact that the subject does.

Examples of pancreatic-cancer-carbohydrate markers include CA19-9, DUPAN-2, Span-1, CA50, CA242, TAG-72, SLX, and STN. Of these, CA19-9 and DUPAN-2, for example, are preferable, and CA 19-9 is more preferable. A pancreatic-cancer-carbohydrate marker for use may be, for example, one or more members selected from the group consisting of CA19-9 and DUPAN-2.

2. Diagnosis of Pancreatic Cancer with Higher Accuracy

When a subject has been determined to have a high likelihood of having pancreatic cancer by the test method including step (2a) according to the present invention, the subject can be diagnosed with pancreatic cancer with higher accuracy by further combining the test method of the present invention with step (3a) of applying a method for diagnosing pancreatic cancer to the subject who has been determined to have a high likelihood of having pancreatic cancer in step (2a) (step (3a)). Additionally, because the test method of the present invention can determine the likelihood of having pancreatic cancer with higher sensitivity, a subject who truly has pancreatic cancer can be diagnosed more reliably as having pancreatic cancer by combining the test method of the present invention with step (3a)(i.e., further reducing the possibility of falsely diagnosing such a subject as not having pancreatic cancer, and the possibility of excluding the subject from diagnosis in step (3a)).

The method for diagnosing pancreatic cancer used in step (3a) is not particularly limited, and various known diagnostic methods can be used. Examples of diagnostic methods include biopsy, PET, CT, ultrasonography, and pancreatic cancer markers (e.g., CA19-9, DUPAN-2, CEA, and CA50). Of these, from the standpoint of diagnosing pancreatic cancer with higher accuracy, biopsy, PET, CT, and ultrasonography, for example, are preferable. These diagnostic methods may be used singly or in a combination of two or more.

3. Treatment of Pancreatic Cancer

When a subject has been determined to have a high likelihood of having pancreatic cancer by the test method including step (2a) according to the present invention, or when a subject has been diagnosed as having pancreatic cancer in step (3a), pancreatic cancer can be treated by (4) further performing pancreatic cancer treatment on the subject who has been determined to have a high likelihood of having pancreatic cancer in step (2a) or the subject who has been diagnosed with pancreatic cancer in step (3a) (step (4)). Additionally, because the test method of the present invention can determine the likelihood of having pancreatic cancer with higher sensitivity, a subject who truly has pancreatic cancer can be treated more reliably by combining the test method of the present invention or a combination of the test method of the present invention and step (3a) with step (4) (i.e., further reducing the possibility of excluding a subject who truly has pancreatic cancer from treatment).

The method for treating pancreatic cancer is not particularly limited, and various known treatment methods can be used. Examples of treatment methods include chemotherapy, surgery, radiation therapy, and immunotherapy. These treatments can be performed in accordance with known methods.

The therapeutic agent for use in chemotherapy is not particularly limited, and various anticancer agents can be used. Examples of anticancer agents include alkylating agents, antimetabolites, microtubule inhibitors, anticancer antibiotics, topoisomerase inhibitors, platinum-containing agents, molecular-targeted agents, hormonal agents, and biologics. Examples of alkylating agents include cyclophosphamide, ifosfamide, nitrosourea, dacarbazine, temozolomide, nimustine, busulfan, melphalan, procarbazine, and ranimustine. Examples of antimetabolites include enocitabine, carmofur, capecitabine, tegafur, tegafur-uracil, tegafur-gimeracil-oteracil potassium, gemcitabine, cytarabine, cytarabine-octophosphate, neralabin, fluorouracil, fludarabine, pemetrexed, pentostatin, methotrexate, cladribine, doxifluridine, hydroxycarbamide, and mercaptopurine. Examples of microtubule inhibitors include alkaloid anticancer agents, such as vincristine, and taxane anticancer agents, such as docetaxel and paclitaxel. Examples of anticancer antibiotics include mitomycin C, doxorubicin, epirubicin, daunorubicin, bleomycin, actinomycin D, aclarubicin, idarubicin, pirarubicin, peplomycin, mitoxantrone, amrubicin, and zinostatin stimalamer. Examples of topoisomerase inhibitors include CPT-11 having topoisomerase I inhibition action, irinotecan, nogitecan, etoposide having topoisomerase II inhibition action, and sobuzoxane. Examples of platinum-containing agents include cisplatin, nedaplatin, oxaliplatin, and carboplatin. Examples of hormonal agents include dexamethasone, finasteride, tamoxifen, astrozole, exemestane, ethinylestradiol, chlormadinone, goserelin, bicalutamide, flutamide, prednisolone, leuprorelin, letrozole, estramustine, toremifene, fosfestrol, mitotane, methyltestosterone, medroxyprogesterone, and mepitiostane. Examples of biologics include interferon $\alpha$, $\beta$, and $\gamma$, interleukin 2, ubenimex, and dried BCG. Examples of molecular-targeted agents include rituximab, alemtuzumab, trastuzumab, cetuximab, panitumumab, imatinib, dasatinib, nilotinib, gefitinib, erlotinib, temsirolimus, bevacizumab, VEGF trap, sunitinib, sorafenib, tocilizumab, bortezomib, gemtuzumab ozogamicin, ibritumomab ozogamicin, ibritumomab tiuxetan, tamibarotene, and tretinoin. Examples of molecular-targeted agents also include, in addition to the molecularly targeted agents listed above, inhibitors targeting angiogenesis, such as human epidermal growth factor receptor 2 inhibitors, epidermal growth factor receptor inhibitors, Bcr-Abl tyrosine kinase inhibitors, epidermal growth factor tyrosine kinase inhibitors, mTOR inhibitors, and vascular endothelial growth factor receptor 2 inhibitors ($\alpha$-VEGFR-2 antibody); tyrosine kinase inhibitors, such as MAP kinase inhibitors; inhibitors targeting cytokines; proteasome inhibitors; and antibody-anticancer drug combinations. These inhibitors also include antibodies.

Examples of typical pancreatic cancer therapeutic agents include gemcitabine, TS-1, erlotinib, a combination drug of gemcitabine and erlotinib, a combination drug of gemcitabine and albumin-bound paclitaxel, and a combination of 4 kinds of drugs (oxaliplatin, levofolinate, irinotecan, and fluorouracil).

## 4. Test Agent for Pancreatic Cancer

The present invention relates to, in one aspect, a test agent for pancreatic cancer containing at least one compound selected from the group consisting of compound 1 containing a 3-sialyllactose carbohydrate structure and compound 2 containing an (LN)3 carbohydrate structure ("the test agent of the present invention"). The following describes this test agent.

The test agent of the present invention examines the likelihood of having pancreatic cancer.

The test agent of the present invention may be in the form of a composition containing at least one compound selected from the group consisting of compound 1 containing a 3-sialyllactose carbohydrate structure and compound 2 containing an (LN)3 carbohydrate structure. The composition may optionally contain other components. Examples of other components include bases, carriers, solvents, dispersants, emulsifiers, buffers, stabilizers, excipients, binders, disintegrants, lubricants, thickeners, moisturizers, coloring agents, flavorings, and chelating agents.

The test agent of the present invention may be in the form of a kit containing at least one compound selected from the group consisting of compound 1 containing a 3-sialyllactose carbohydrate structure and compound 2 containing an (LN)3 carbohydrate structure. The kit may contain instruments, reagents, etc. for use in performing the test method of the present invention.

Examples of instruments include test tubes, microtiter plates, agarose particles, latex particles, purification columns, epoxy-coated glass slides, and gold colloid-coated glass slides.

Examples of reagents include labeled antibodies, and reference samples (positive control, negative control).

A variety of commercially available antibodies can be used for such a labeled antibody according to the isotype of the anti-carbohydrate antibody of the present invention to be detected.

The anti-carbohydrate antibody of the present invention is used for a reference sample. The anti-carbohydrate antibody of the present invention is obtained, for example, by preparing hybridomas that produce an antibody that binds to 3-sialyllactose carbohydrate or (LN)3 carbohydrate using hybridoma technology, and purifying the culture supernatant.

More specifically, the anti-carbohydrate antibody of the present invention can be obtained, for example, as follows. First, lymphocytes prepared from lymph nodes or spleen are fused with myeloma cells such as SPM 4-0, and cultured. Four weeks after fusion, the supernatant of the hybridoma culture is obtained, and a hybridoma in the culture supernatant that reacts with 3-sialyllactose carbohydrate or (LN)3 carbohydrate is selected. The anti-carbohydrate antibody of the present invention can be obtained from the supernatant prepared by culturing a large amount of the selected hybridoma by salting-out and affinity purification using a Protein G column etc.

## Examples

The present invention is described in detail below based on Examples; however, the present invention is not limited to these Examples.

Reference Example 1: Serum anti-carbohydrate antibody level

(a) Preparation of carbohydrate array

3-Sialyllactose-PAA (produced by GlycoTech Corporation, catalog number: 08-038) and Galβ1 → 3GlcNAcβ1 → 3Galβ1 → 4GlcNAc-PAA (produced by GlycoTech Corporation, catalog number: 08-096) were separately diluted in a solution (hereinafter sometimes referred to as "spot solution") containing equivalent amounts of purified water and a spotting solution (produced by Matsunami Glass Ind., Ltd., catalog number: DSP 0050), thereby preparing 0.5 mg/mL diluted solutions.

Subsequently, 1 μL of the 3'-sialyllactose-PAA-containing diluted solution and 1 μL of the Galβ1 → 3GlcNAcβ1 → 3Galβ1 → 4GlcNAc-PAA-containing diluted solution were separately added dropwise to a glass slide (produced by Schott AG, catalog number: 1066643) coated with epoxy silane so that each droplet had a diameter of 1 mm, and allowed to stand at room temperature for 16 hours.

Thereafter, each glass slide was washed three times with a washing solution (25 mM Tris-HCl (pH 7.4), 0.8% NaCl, 1% Triton-X 100), thereby removing excess spot solution. A blocking solution (25 mM Tris-HCL (pH 7.4), 0.8% NaCl, 1% Triton-X 100, 4% BSA) was then added and allowed to stand at room temperature for 1 hour, thereby protecting the unreacted epoxy group of the epoxy silane. Thus, a carbohydrate array containing 3'-sialyllactose-PAA and a carbohydrate array containing Galβ1 → 3GlcNAcβ1 → 3Galβ1 → 4GlcNAc-PAA were prepared. These carbohydrate arrays were refrigerated until use.

(b) Measurement of anti-carbohydrate antibody levels in serum of healthy subject specimens and pancreatic cancer patient specimens

As biological samples, serum of 25 healthy subject specimens and 16 pancreatic cancer patient specimens (stage 1: one specimen, stage 2: six specimens, stage 3: two specimens, and stage 4: seven specimens) was obtained from two hospitals through Wako Pure Chemical Industries.

1 μL of each serum specimen was diluted 100-fold with a specimen dilution solution (25 mM Tris-HCL (pH 7.4), 0.8% NaCl, 1% Triton-X 100, 1 mM $MnCl_2$, 1 mM $CaCl_2$, 1% BSA) . 80 μL of the resulting diluent (containing the equivalent of 0.8 μL of serum) was added to each of the carbohydrate arrays prepared in Reference Example 1(a), allowed to stand at room temperature for 3 hours, and then washed once with a washing solution.

Subsequently, a Cy3 (registered tradename) fluorochrome-labeled goat anti-human IgG antibody (produced by Jackson ImmunoResearch Laboratories Inc.) was added to each of the carbohydrate arrays containing the serum diluted with the specimen dilution solution, allowed to stand at room temperature for one hour, and then washed three times with a washing solution. Fluorescence images were obtained using an FLA-5100 fluorescent image analyzer.

The fluorescence intensity at each spot was measured using Image J (http://imagej.nih.gov/ij/) to obtain the fluorescence intensity value of each serum.

Further, each serum specimen was diluted 200,901-fold using an Immunoglobulin G ELISA kit (produced by Immunodiagnostik, catalog number: K6510A), and the amount of human IgG contained in the equivalent of 0.8 μL of serum was measured.

The measurement values were substituted into the following equation to calculate the serum anti-carbohydrate antibody (anti-3-sialyllactose carbohydrate antibody or anti-(LN)3 carbohydrate antibody) concentration (anti-carbohydrate antibody level).

(anti-carbohydrate antibody level) = (fluorescence intensity value)/(amount of human IgG contained in the equivalent of 0.8 μL of serum)

Consequently, as shown in Fig. 1, the anti-carbohydrate antibody levels in the serum of the pancreatic cancer patients were clearly lower than those of the healthy subjects. This indicated that the development of pancreatic cancer was correlated with the anti-carbohydrate antibody level.

Example 1: Examination of pancreatic cancer using anti-3-sialyllactose carbohydrate antibody level or anti-(LN)3 carbohydrate antibody level as an indicator

The anti-carbohydrate antibody levels in the serum of 16 pancreatic cancer patient specimens (stage 1: one specimen, stage 2: six specimens, stage 3: two specimens, and stage 4: seven specimens) obtained in Reference

Example 1(b) were individually compared with the cutoff value. Specimens having a value lower than the cutoff value were determined to have pancreatic cancer (positive).

The cutoff value was the 50th percentile of the anti-carbohydrate antibody levels of 25 healthy subject specimens obtained in Reference Example 1. The cutoff value of anti-3-sialyllactose carbohydrate antibody was 63.16 (fluorescence intensity value/amount of IgG (pg)), and the cutoff value of anti-(LN)3 carbohydrate antibody was 13.09 (fluorescence intensity value/amount of IgG ($\mu$g)).

Fig. 2 shows graphs representing the results according to each pancreatic cancer stage.

The rate of early-stage (stages 1 and 2) pancreatic cancer patient specimens that were determined to be positive (to have a high likelihood of having pancreatic cancer) was 6/7 when the anti-3-sialyllactose carbohydrate antibody level or the anti-(LN)3 carbohydrate antibody level was used.

Comparative Example 1: Examination of pancreatic cancer using a known marker as an indicator

As in Reference Example 1, the concentrations of CA19-9 in the serum of 16 pancreatic cancer patient specimens (stage 1: one specimen, stage 2: six specimens, stage 3: two specimens, and stage 4: seven specimens) were measured using a commercially available kit (produced by ALPCO, catalog number: 25-199HU-E01).

The obtained CA19-9 concentrations were compared with the cutoff value, and specimens having a value higher than the cutoff value were determined to have pancreatic cancer (positive).

The cutoff value used here was a typically used value (37 U/mL).

Fig. 2 shows graphs representing the results according to each pancreatic cancer stage.

The rate of early-stage (stages 1 and 2) pancreatic cancer patient specimens that were determined to be positive (to have a high likelihood of having pancreatic cancer) was 3/7.

The results of Example 1 and Comparative Example 1 indicated that the development of early-stage (stages 1 and 2) pancreatic cancer was determined with high accuracy when the anti-3-sialyllactose carbohydrate antibody level or the anti-(LN)3 carbohydrate antibody level was used as compared to when the CA19-9 concentration was used.

Reference Example 2: Serum anti-3-sialyllactose carbohydrate antibody concentration

(a) Preparation of calibration curve

0.5 mg of biotin-labeled 3'-sialyllactose-PAA was dissolved in 0.8 mL of PBS (phosphate-buffered saline). The entire amount of the mixture was added to 0.8 mL of streptavidin-agarose resin (produced by Thermo Fisher Scientific, catalog number: 20353), and reacted under stirring at 4°C for 12 hours. After the reaction, the resin was placed in a Poly-Prep (registered tradename) empty column (produced by BIO-RAD Laboratories, Inc., catalog number: 731-1550), thereby preparing a 3'-sialyllactose-PAA immobilized column. 50 mg of lyophilized IgG derived from healthy subject serum (Wako Pure Chemical Industries, code number: 289-95341, lot number: WDN 9152) was dissolved in PBS containing 5 mL of 0.1% Tween-20, and the resultant was added to the 3'-sialyl lactose-PAA immobilized column. Thereafter, the column was sealed and reacted under stirring at 4°C for 12 hours. After the reaction, the column was released, and a non-adsorbed component was eluted. Additionally, after the column was washed with 4 mL of PBS, 1 mL of a 3 mM 3'-sialyllactose (produced by Tokyo Chemical Industry Co., Ltd., catalog number: S0885) PBS(-) solution was added. The column was then sealed and reacted under stirring at 4°C for 3 hours. After the reaction, the column was released, and an anti-3-sialyllactose carbohydrate antibody (human IgG) was eluted. Furthermore, after 4 mL of a 3 mM 3'-sialyllactose PBS(-) solution was added to the column to collect an eluate, the eluate was mixed with the anti-3-sialyllactose carbohydrate antibody eluate. The resulting eluate was subjected to ultrafiltration using Amicon (registered tradename) Ultra-15 mL (NMWL of 50 kDa, produced by Millipore, code number: UFC 905024), and the concentration of IgG was measured. Thereafter, IgG was taken as a standard product of anti-3-sialyllactose carbohydrate antibody.

100 $\mu$L of 10 $\mu$g/ml 3'-sialyllactose-PAA was added to each well of a MaxiSorp 96-well plate (produced by Thermo Fisher Scientific, catalog number: 439454), and allowed to stand at room temperature for 16 hours. Subsequently, each well was washed three times with 300 $\mu$L of PBS (washing solution) containing 1% Triton-X 100. 200 $\mu$L of PBS containing 5% BSA and 1% Triton-X 100 was added thereto and allowed to stand at room temperature for 1 hour. After each well was washed with 300 $\mu$l of washing solution, 100 $\mu$L of anti-3-sialyllactose carbohydrate antibody solution (0, 6.25, 12.5, 25, 50, 100, or 250 ng/ml) was added thereto and allowed to stand at room temperature for two hours. Thereafter, each well was washed five times with 300 $\mu$L of washing solution, and 100 $\mu$L of peroxidase-labeled goat anti-human IgG antibody (produced by SeraCare Life Sciences, catalog number: 04-10-06) diluted 200-fold was added and allowed to stand at room temperature for one hour. Subsequently, each well was washed three times with 300 $\mu$L of washing solution, and 100 $\mu$L of a peroxidase substrate solution (Peroxidase Substrate Solutions A and B (code numbers: 5120-0034 and 50-65-00) produced by SeraCare Life Sciences were mixed in equal amounts) was added thereto and allowed to stand at room temperature for 20 minutes. After the reaction, 100 $\mu$L of a reaction-stopping agent

(SeraCare Life Sciences, code number: 5150-0017) was added, and the absorbance of each well at 450 nm was measured using a microplate reader. A calibration curve in which the vertical axis represents absorbance and the horizontal axis represents the amount of anti-3-sialyllactose carbohydrate antibody solution was prepared.

As a result, a high correlation ($R^2$=0.9949) between anti-3-sialyllactose carbohydrate antibody concentration and absorbance was confirmed, and the anti-3-sialyllactose carbohydrate antibody concentration was calculated according to the following formula:

(anti-3-sialyllactose carbohydrate antibody concentration) = 372.27 x (absorbance)

(b) Measurement of anti-3-sialyllactose carbohydrate antibody concentration in serum of healthy subject specimens and pancreatic cancer patient specimens

As biological samples, serum of 13 healthy subject specimens and 55 pancreatic cancer patient specimens (stage 1: 15 specimens, stage 2: 15 specimens, stage 3: 11 specimens, and stage 4: 14 specimens) was obtained from a hospital in the United States through Wako Pure Chemical Industries.

Each serum specimen was diluted 750-fold with a specimen dilution solution (1% BSA and 1% Triton-X 100), and 100 μL of the diluted serum was added to a MaxiSorp 96-well plate on which 3'-sialyllactose-PAA had been adsorbed beforehand. Absorbance at 450 nm was measured in the same manner as in the method described in Reference Example 2(a), and the anti-3-sialyllactose carbohydrate antibody concentration in each serum was calculated based on the calibration curve.

Consequently, as shown in Fig. 3, the anti-3-sialyllactose carbohydrate antibody concentrations in the serum of the pancreatic cancer patient specimens were clearly lower than those of the healthy subject specimens. This indicated that the development of pancreatic cancer was correlated with the serum anti-3-sialyllactose carbohydrate antibody concentration.

Example 2: Examination of pancreatic cancer using anti-3-sialyllactose carbohydrate antibody concentration as an indicator

Based on the results of Reference Example 2, an ROC curve in which the vertical axis represents sensitivity (positive rate) (%) and the horizontal axis represents the value obtained by subtracting specificity (%) from 100% (100% - specificity (%)) (false positive rate) was made using the medical statistical software GraphPad Prism, and shown in Fig. 4. The area under the curve (AUC) showed a high value of 0.87, and the cutoff value obtained using the Youden's index as an indicator was 50.08 pg/mL (sensitivity = 76.36%, specificity = 92.31%). Sensitivity here indicates the percentage of pancreatic cancer (stages 1, 2, 3 and 4) patient specimens that are determined to be positive, and specificity indicates the percentage of healthy subject specimens that are determined to be negative. The results of determination based on the above values indicated that the sensitivity and specificity in this examination method were 76% and 92%, respectively.

Example 3: Examination of early-stage pancreatic cancer in combination with the use of known marker

As in Reference Example 2(b), the concentrations of CA19-9 in the serum of 13 healthy subject specimens and 55 pancreatic cancer patient specimens (stage 1: 15 specimens, stage 2: 15 specimens, stage 3: 11 specimens, and stage 4: 14 specimens) were measured using a commercially available kit (produced by Abnova Corporation, catalog number: KA0207). From the obtained measurement values, the cutoff value was calculated using Youden's index as an indicator and found to be 24.80 U/mL. With respect to early-stage (stages 1 and 2) pancreatic cancer patient specimens, the number of cases determined to be negative (the number of false negative cases; to have a high likelihood of not having pancreatic cancer) was counted based on this value. On the other hand, cases that were determined to be positive (to have a high likelihood of having pancreatic cancer) in either the determination of development of pancreatic cancer using the anti-3-sialyllactose carbohydrate antibody concentration as an indicator (Example 2) or the determination of development of pancreatic cancer using CA19-9 as an indicator, or both, were determined to be "positive." The number of cases that were determined to be negative (to have a high likelihood of not having pancreatic cancer) in both the determination of development of pancreatic cancer using the anti-3-sialyllactose carbohydrate antibody concentration as an indicator and the determination of development of pancreatic cancer using CA19-9 as an indicator was counted as the number of false negative cases.

Table 1 shows the results. With respect to early-stage (stages 1 and 2) pancreatic cancer patient specimens, the number of cases that were determined to be negative (the number of false negative cases; to have a high likelihood

of not having pancreatic cancer) in both the determination of development of pancreatic cancer using the anti-3-sialyllactose carbohydrate antibody concentration as an indicator and the determination of development of pancreatic cancer using CA19-9 as an indicator was smaller than the number of cases that were determined to be false negatives in the examination using CA19-9 alone as an indicator.

Table 1

| Indicator | Number of false negative cases (stage 1) | Number of false negative cases (stage 2) |
|---|---|---|
| CA19-9 | 4 | 1 |
| CA19-9 + anti-3-sialyllactose carbohydrate antibody | 1 | 0 |

Industrial Applicability

The test method of the present invention can determine the likelihood of early-stage pancreatic cancer development with high accuracy, as compared to conventional test methods. By measuring the anti-carbohydrate antibody level of the present invention, the progression of pancreatic cancer and the efficacy of anticancer drugs can be monitored.

## Claims

1. A method for examining the likelihood of having pancreatic cancer,
the method comprising (1) measuring the amount or concentration of at least one anti-carbohydrate antibody selected from the group consisting of anti-3-sialyllactose carbohydrate antibody and anti-(LN)3 carbohydrate antibody in a biological sample obtained from a subject,
wherein the anti-3-sialyllactose carbohydrate antibody is an antibody capable of binding to a carbohydrate represented by Neu5Acα (2→3) Galβ (1→4) Glc or an antibody recognizing the carbohydrate, and
the anti-(LN)3 carbohydrate antibody is an antibody capable of binding to a carbohydrate represented by Galβ1 → 3GlcNAcβ1 → 3Galβ1 → 4GlcNAc or an antibody recognizing the carbohydrate.

2. The method according to claim 1, wherein step (1) comprises

    (1a) bringing at least one compound selected from the group consisting of compound 1 containing a 3-sialyllactose carbohydrate structure and compound 2 containing an (LN)-3 carbohydrate structure into contact with the biological sample; and
    (1b) measuring the amount of an anti-carbohydrate antibody bound to the at least one compound.

3. The method according to claim 1 or 2, further comprising
(2a) determining that the subject has a high likelihood of having pancreatic cancer when the value of the amount or concentration of the at least one anti-carbohydrate antibody measured in step (1) is less than or equal to a predetermined cutoff value.

4. The method according to claim 3, wherein the cutoff value is an average value, a percentile value, or a minimum value of the amounts or concentrations of the at least one anti-carbohydrate antibody in biological samples obtained from subjects who are not affected by pancreatic cancer.

5. The method according to claim 1 or 2, further comprising

    (2c) tentatively determining that the subject has a high likelihood of having pancreatic cancer when the value of the amount or concentration of the at least one anti-carbohydrate antibody measured in step (1) is less than or equal to a predetermined cutoff value;
    (3c) measuring the amount or concentration of a pancreatic-cancer-carbohydrate marker in the biological sample, and tentatively determining that the subject has a high likelihood of having pancreatic cancer when the value of the measured amount or concentration of the pancreatic-cancer-carbohydrate marker is equal to or more than a predetermined cutoff value; and
    (3d) determining that the subject has a high likelihood of having pancreatic cancer on the basis of the tentative

determination in step (2c) and the tentative determination in step (3c).

**6.** The method according to claim 5, wherein step (3d) comprises determining that the subject has a high likelihood of having pancreatic cancer when the subject has been tentatively determined to have a high likelihood of having pancreatic cancer in either the tentative determination in step (2c) or the tentative determination in step (3c), or both.

**7.** The method according to claim 5 or 6, wherein the cutoff value for the amount or concentration of the at least one anti-carbohydrate antibody is an average value, a percentile value, or a minimum value of the amounts or concentrations of the at least one anti-carbohydrate antibody in biological samples obtained from subjects who are not affected by pancreatic cancer.

**8.** The method according to any one of claims 5 to 7, wherein the pancreatic-cancer-carbohydrate marker is at least one member selected from the group consisting of CA19-9 and DUPAN-2.

**9.** The method according to claim 8, wherein the pancreatic-cancer-carbohydrate marker is CA19-9.

**10.** The method according to any one of claims 1 to 9, wherein the at least one anti-carbohydrate antibody is IgG.

**11.** The method according to any one of claims 1 to 10, wherein the biological sample is a body fluid or a sample derived from a body fluid.

**12.** The method according to claim 11, wherein the body fluid is at least one member selected from the group consisting of whole blood, serum, and plasma.

**13.** The method according to any one of claims 1 to 12, wherein the pancreatic cancer to be detected is at a stage of 0 to 2.

**14.** A method for measuring the amount or concentration of an anti-carbohydrate antibody in a subject,
the method comprising (1) measuring the amount or concentration of at least one anti-carbohydrate antibody selected from the group consisting of anti-3-sialyllactose carbohydrate antibody and anti-(LN)3 carbohydrate antibody in a biological sample obtained from a subject,
wherein the anti-3-sialyllactose carbohydrate antibody is an antibody capable of binding to a carbohydrate represented by Neu5Ac$\alpha$ (2$\rightarrow$3) Gal$\beta$ (1$\rightarrow$4) Glc or an antibody recognizing the carbohydrate, and
the anti-(LN)3 carbohydrate antibody is an antibody capable of binding to a carbohydrate represented by Gal$\beta$1 $\rightarrow$ 3GlcNAc$\beta$1 $\rightarrow$ 3Gal$\beta$1 $\rightarrow$ 4GlcNAc or an antibody recognizing the carbohydrate.

**15.** The method according to claim 14, wherein in step (1), the amount or concentration of only one anti-carbohydrate antibody selected from the group consisting of anti-3-sialyllactose carbohydrate antibody and anti-(LN)3 carbohydrate antibody is measured as the anti-carbohydrate antibody.

**16.** The method according to claim 14 or 15, wherein step (1) comprises
immobilizing on a solid phase at least one compound selected from the group consisting of compound 1 containing a 3-sialyllactose carbohydrate structure and compound 2 containing an (LN)3 carbohydrate structure,
bringing the at least one compound immobilized on the solid phase into contact with a biological sample obtained from a subject,
washing with a solution containing trishydroxymethylaminomethane and/or an ether-type nonionic surfactant the solid phase on which the at least one compound that has been brought into contact with the biological sample is immobilized,
adding a blocking solution that contains bovine serum albumin and that further contains trishydroxymethylaminomethane and/or an ether-type nonionic surfactant to the washed solid phase, and
measuring the amount or concentration of an antibody bound to the at least one compound immobilized on the solid phase.

**17.** A test agent for pancreatic cancer comprising at least one compound selected from the group consisting of compound 1 containing a 3-sialyllactose carbohydrate structure and compound 2 containing an (LN)3 carbohydrate structure, wherein the 3-sialyllactose carbohydrate structure is represented by Neu5Ac$\alpha$ (2$\rightarrow$3) Gal$\beta$ (1$\rightarrow$4) Glc, and the (LN)3 carbohydrate structure is represented by Gal$\beta$1 $\rightarrow$ 3GlcNAc$\beta$1 $\rightarrow$ 3Gal$\beta$1 $\rightarrow$ 4GlcNAc.

**18.** At least one compound selected from the group consisting of compound 1 containing a 3-sialyllactose carbohydrate

structure represented by Neu5Acα (2→3) Galβ (1→4) Glc and compound 2 containing an (LN)3 carbohydrate structure represented by Galβ1 → 3GlcNAcβ1 → 3Galβ1→4GlcNAc, the at least one compound being for use as a test agent for pancreatic cancer for the likelihood of having pancreatic cancer.

**19.** Use of a compound in examining the likelihood of having pancreatic cancer, the compound being at least one compound selected from the group consisting of compound 1 containing a 3-sialyllactose carbohydrate structure represented by Neu5Acα (2→3) Galβ (1→4) Glc and compound 2 containing an (LN)3 carbohydrate structure represented by Galβ1 → 3GlcNAcβ1 → 3Galβ1 → 4GlcNAc.

**20.** Use of a compound in producing a test agent for examining the likelihood of pancreatic cancer, the compound being at least one compound selected from the group consisting of compound 1 containing a 3-sialyllactose carbohydrate structure represented by Neu5Acα (2→3) Galβ (1→4) Glc and compound 2 containing a (LN)3 carbohydrate structure represented by Galβ1 → 3GlcNAcβ1 → 3Galβ1 → 4GlcNAc.

Fig. 1

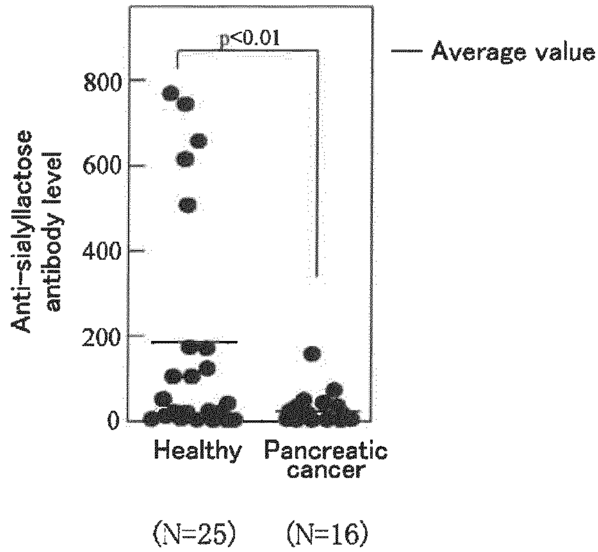
p<0.01
Average value
Anti-sialyllactose antibody level
800
600
400
200
0
Healthy
Pancreatic cancer
(N=25)
(N=16)

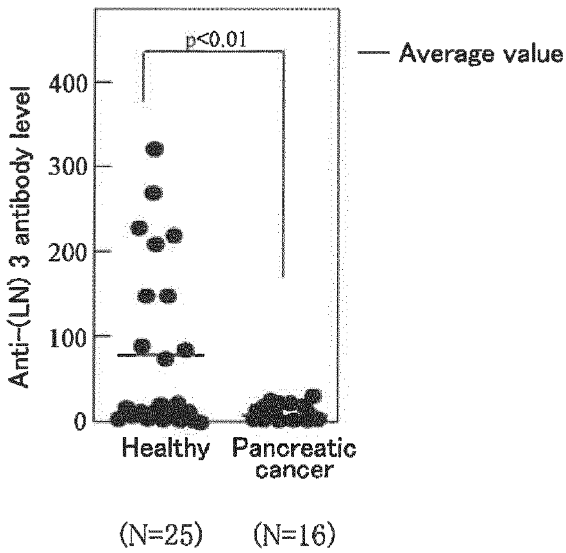
p<0.01
Average value
Anti-(LN) 3 antibody level
400
300
200
100
0
Healthy
Pancreatic cancer
(N=25)
(N=16)

Fig. 2

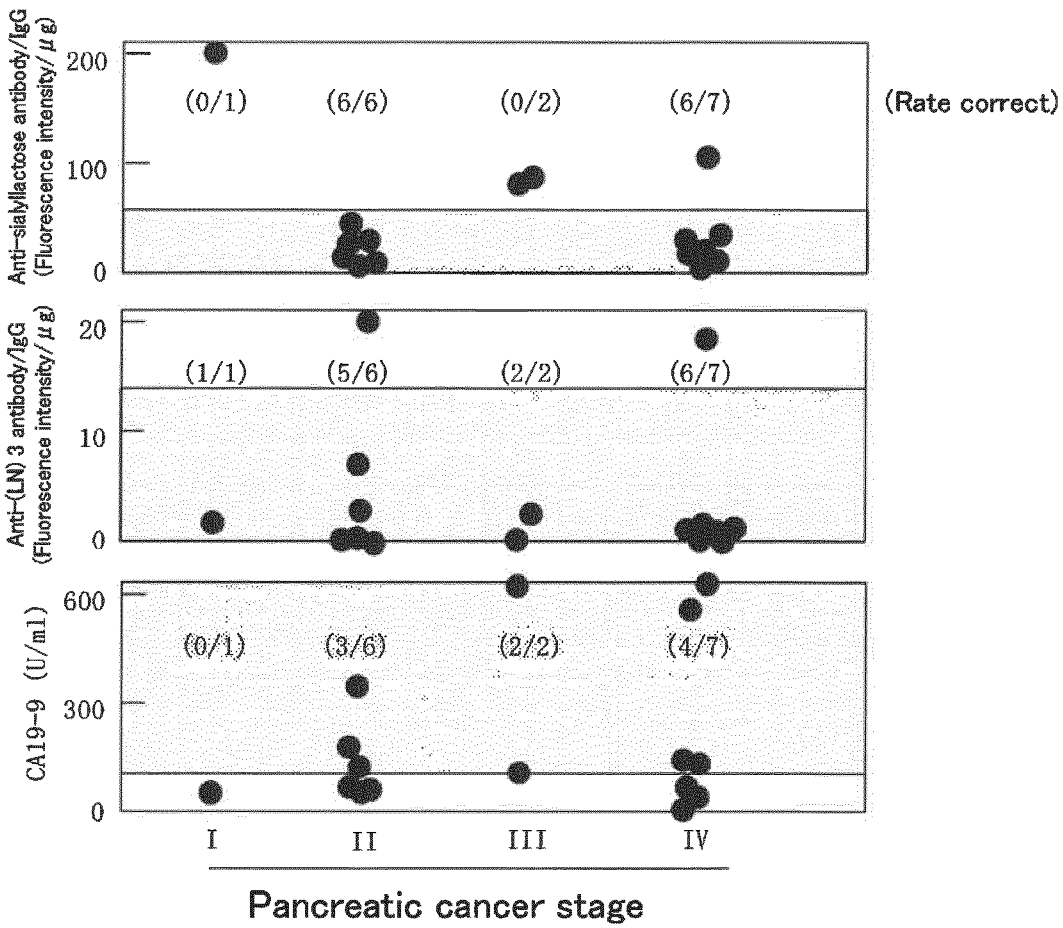
Anti-sialyllactose antibody/IgG (Fluorescence intensity/μg)
200
100
0
(0/1)
(6/6)
(0/2)
(6/7)
(Rate correct)
Anti-(LN) 3 antibody/IgG (Fluorescence intensity/μg)
20
10
0
(1/1)
(5/6)
(2/2)
(6/7)
CA19-9 (U/ml)
600
300
0
(0/1)
(3/6)
(2/2)
(4/7)
I
II
III
IV
Pancreatic cancer stage

Fig. 3

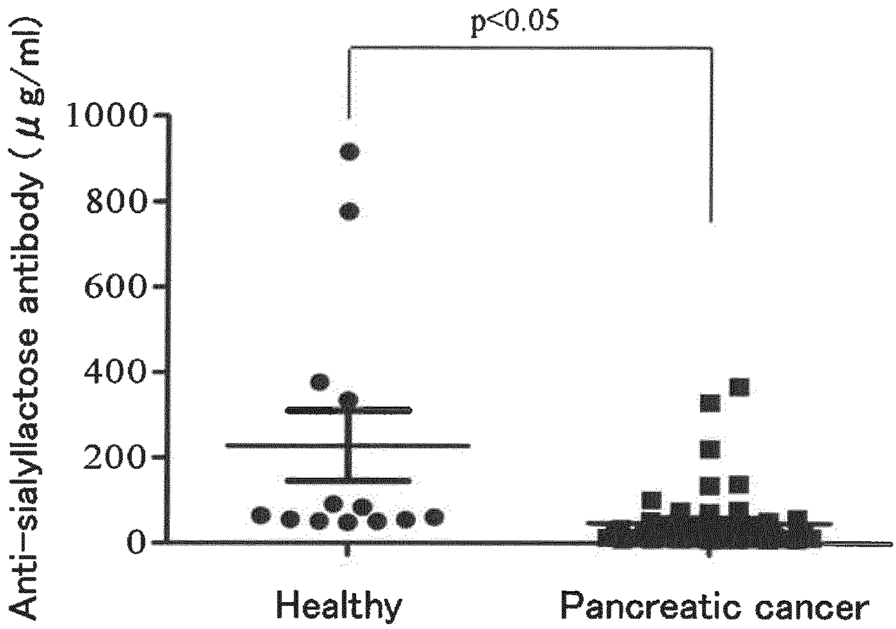
p<0.05
Anti-sialyllactose antibody (μg/ml)
1000
800
600
400
200
0
Healthy
Pancreatic cancer

Fig. 4

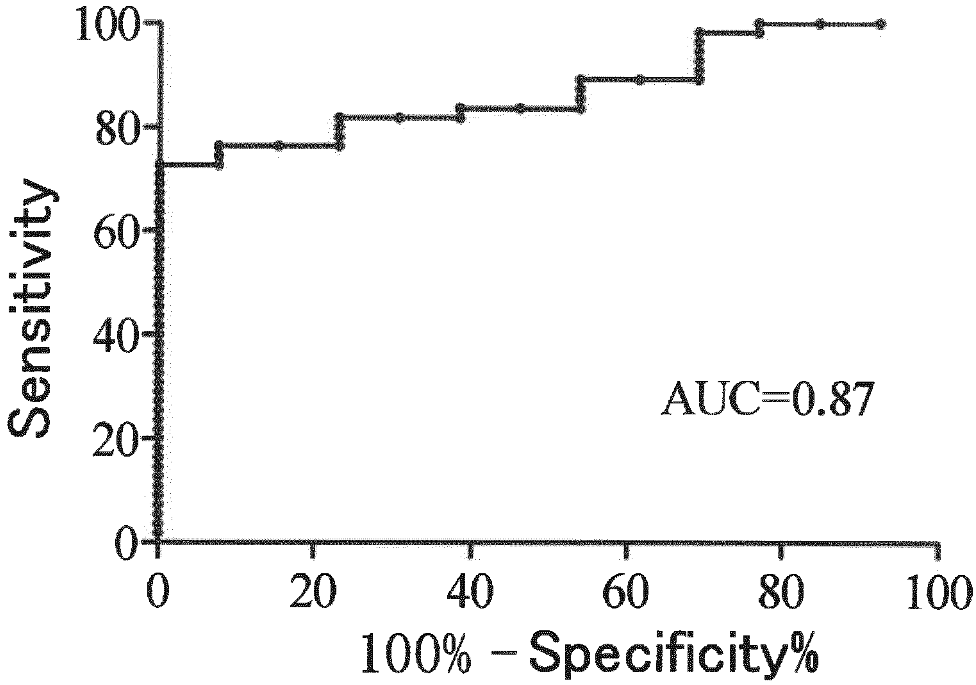
100
80
60
40
20
0
Sensitivity
AUC=0.87
0
20
40
60
80
100
100% - Specificity%

## INTERNATIONAL SEARCH REPORT

International application No. PCT/JP2018/003394

A. CLASSIFICATION OF SUBJECT MATTER
Int.Cl. G01N33/574(2006.01)i, G01N33/564(2006.01)i

According to International Patent Classification (IPC) or to both national classification and IPC

B. FIELDS SEARCHED

Minimum documentation searched (classification system followed by classification symbols)
Int.Cl. G01N33/574, G01N33/564

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/MEDLINE/EMBASE/BIOSIS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| A | JP 2017-504011 A (METANOMICS HEALTH GMBH) 02 February 2017, entire text, all drawings, etc. & US 2016/0313338 A1, entire text, all drawings, etc. & WO 2015/091962 A1 & EP 3084443 A1 | 1-20 |
| A | JP 2016-205827 A (TOSOH CORPORATION) 08 December 2016, entire text, all drawings, etc. (Family: none) | 1-20 |
| A | JP 2005-069926 A (JSR CORPORATION) 17 March 2005, entire text, particularly, paragraph [0014], etc. (Family: none) | 1-20 |

☒ Further documents are listed in the continuation of Box C.　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>26.04.2018 | Date of mailing of the international search report<br>15.05.2018 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

INTERNATIONAL SEARCH REPORT

International application No.
PCT/JP2018/003394

C (Continuation). DOCUMENTS CONSIDERED TO BE RELEVANT

| Category* | Citation of document, with indication, where appropriate, of the relevant passages | Relevant to claim No. |
|---|---|---|
| A | JP 2010-235564 A (KATO, Junji) 21 October 2010, entire text, all drawings, etc. (Family: none) | 1-20 |
| A | JP 2007-298334 A (MITSUBISHI CHEMICAL CORPORATION) 15 November 2007, entire text, etc. (Family: none) | 1-20 |
| A | JP 2013-006801 A (RITSUMEIKAN) 10 January 2013, entire text, all drawings, etc. (Family: none) | 1-20 |
| A | WO 2013/147213 A1 (DAIICHI SANKYO CO., LTD.) 03 October 2013, entire text, etc. & US 2015/0056189 A1 & EP 2832857 A1 | 1-20 |
| A | WO 2011/105544 A1 (OSAKA PREFECTURAL HOSPITAL ORGANIZATION) 01 September 2011, entire text, etc. & JP 2013-100233 A | 1-20 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ERXI W. ; SHUANG Z. ; KRUTTIKA B. ; QINGYONG M.** CA 19-9 and pancreatic cancer. *Clin Adv Hematol Oncol.,* 2013, vol. 11 (1), 53-55